# EUROPEAN PATENT SPECIFICATION

(11) **EP 1 245 244 B1**
(45) Date of publication and mention of the grant of the patent: **08.08.2007**
(21) Application number: 02007007.4
(22) Date of filing: 20.06.1997
(51) Int. Cl.: A61M 11/06, B05B 7/00

(54) **Dispensing system**
Abgabevorrichting
Système de distribution

(30) Priority: 20.06.1996 GB 9612968
(43) Date of publication of application: 02.10.2002
(62) Divisional of application: 97927296.0
(73) Proprietor: Profile Drug Delivery Limited, Bognor Regis, West Sussex PO22 9SL (GB)
(72) Inventor: Denyer, Jonathan Dh., Chichester West Sussex PO19 3PW (GB); Dyche, Anthony, Hampshire PO11 0ND (GB); Basista, Jacek L., Tunbridge Wells Kent TN4 9QT (GB)
(74) Representative: Wright, Howard Hugh Burnby

(56) References cited:
- EP-A- 0 519 742
- WO-A-95/11714
- WO-A-96/13290
- GB-A- 1 568 808
- US-A- 3 658 059
- US-A- 4 558 710

## Description

This invention relates to a dispensing system, in particular one which may be of use for example in the dispensing of small quantities of medicament, where the medicament is required to be dispensed in aerosol form.

It is known for dosing systems which supply a nebulized substance to contain a metering system. For example, in GB 1,568,808, (Rosenthal) there is described a metering system for supplying a nebulized substance for patient inhalation comprising a nebulizing means, a detecting means for detecting the initiation of the patient's inhalation, an adjustable timing means for adjusting a timed operation, and a valve means which is controlled by the timing means, in order to provide a controlled dosage of nebulized substance.

The preamble of claim 1 is based on the disclosure of this document.

However, for this system to work and provide a precise dose of medicament, it is necessary that the system utilises a calibrated nebulizer which has a precise rate of output against time. Commercially available nebulizers have a wide range of outputs against time. In addition, the calibration must remain constant over the use of the nebulizer, and the nebulizer must be connected to the dosing device in such a way that the calibration of the nebulizer is valid and recognised when operated with the dispensing device. This is particularly important in relation to the length of tube between the valve and the nebulizer.

Also, it is noted that this application describes no teaching of a device which is in any way calibrated for use with nominated drugs. This may have been due to the fact that the apparatus embodied in this application is designed for provocation testing, which is generally carried out in a laboratory, and is used to deliver pulses of aerosol into a patient's airway in order to determine their reactivity to allergens. The apparatus is not designed as one for a patient to take home, and to deliver precise doses of medicament on a day to day basis.

In addition to the above application, GB 2,164,569 (Etela-Hameen Kauhkovammayhdiatys RY (Finland)) describes a similar system to that described above, which is also designed for provocation testing, except that it has an additional atomizing starting time control, which is used for selecting the atomizing starting moment to coincide with the beginning of the inspiration phase, which is found by examination to be favourable for a particular patient.

EP 519,742 (DeVilbiss Healthcare Inc) describes a medical nebulizer control system which has a three way valve. In the embodiment, one part of the valve is connected to a pressure sensor, and another is connected to a compressed air supply. The control system within this apparatus uses the supply tube to the nebulizer to detect the patient's breathing pattern. When it detects inhalation, it switches the valve over to the compressed air supply, which then drives the nebulizer for a predetermined period of time, irrespective of whether the patient has continued to inhale or not. This device also suffers from the problems of other nebulisers in that there is a long length of tube between the control box and the nebuliser. In addition the nebuliser can be supplied from any manufacturer, and hence its calibration is not tied into the device, thereby causing variable amounts of medicament to be dispensed.

According to the present invention, there is provided a product dispensing system comprising a calibrated nebuliser having a nebuliser jet, a mouthpiece, means for sourcing compressed air, a manifold for distributing the compressed air in at least one direction, a port, and a valve means for controlling the manifold and thereby the flow of compressed air to the port, the manifold and the port being linked by a length of tube, characterised by a nebuliser accommodation means which is accessed by the port, by a sensor for detecting a patient's breathing to indicate the rate of inhalation of the patient and by means for determining the dose dispensed, the dose determined on the basis of the output rate of the medicament of the nebuliser, and the duration of the delivery of the medicament, the dose determining means serving to adjust the value of the output rate used in calculating the dose on the basis of the rate of inhalation of the patient detected by the sensor.

Preferably, the internal volume of the tube from the manifold outlet to the nebuliser jet is less than 0.5 ml.

Conveniently, the dispensing system according to the invention is utilised in conjunction with a small air compressor as the source of compressed air, which has a flow rate in the region of 1-2 litres per minute.

By "calibrated" in this instance is meant that the dosage rate for the nebuliser has already been established, and the dosimeter programmed accordingly, so as to provide a known dosage rate for a given medicament. It is envisaged that a given dosimeter will have one or more dedicated nebulizers.

In preferred embodiments of the invention, the dispensing system additionally comprises a switch means, which is responsive as to whether compressed air is flowing in the system.

It is another preferred aspect of the invention that it may comprise a dispensing system comprising a dosimeter which has been pre-programmed to dose one or more medicaments according to predetermined dosage profiles.

The dispensing system according to the invention is conveniently one which is capable of being pre-programmed to dose one or more different types of drugs, which may need to be dosed according to different profiles. Methods of pre-programming the dispensing system in such a fashion will be familiar to those skilled in the art. In addition, the nebulizer and dosimeter according to the invention are calibrated to determine the dosage of drug as described in EP 587,380 (Medic-Aid Limited), the contents of which are incorporated herein by reference.

The dispensing system according to the invention incorporates a nebulizer and a mouthpiece, by which nebulized medicament can actually be delivered to the patient. The dispensing system also additionally comprises a pressure sensor, which is capable in use of detecting a pressure drop in the apparatus in response to the patient's breathing, and then delivering a pulse of nebulized medicament into the mouthpiece. In such instances, the dose of medicament can be calculated by the known rate of output against time for the drug selected, and the sum of all nebulizer pulses which the system has delivered, in ways known to those skilled in the art, as referred to above. Such ways may typically include performing clinical trials on the drug to determine appropriate dosages, and programming these electronically into the dispensing system.

It is to be understood that the manifold in a dispensing apparatus according to the invention can, in fact, be an integral part of the valve means, and need not be a separately discreet entity. In certain embodiments, the manifold is a shaped block with internal galleys, for example, made of plastics materials, and its presence allows the tubes to be connected to the ports on the valve. The ports on the valve may be too close together to accommodate the connection of tubes directly, and a manifold increases the space between them in other embodiments it may be necessary for the manifold to distribute compressed air in at least two different directions.

The dispensing system according to the invention can operate from a variety of different compressed air sources, such as a continuous air supply at a rate of around 6 litres per minute. This can typically be provided either from a pressurized cylinder such as those used in hospitals, or can be generated from a conventional air compressor.

In an alternative and preferred configuration, the dispensing system can be operated with a lightweight compressor system, which typically generates a low flow rate (in the order of 1-2 litres per minute, preferably 1.5 litres per minute), in conjunction with an accumulator. Such a combination of low flow rate compressor and accumulator allows the dispensing system to produce pulses of compressed air to a nebulizer in the dispensing system with the flow around 6 litres per minute, where the flow from the compressor is matched to the mean flow through the nebulizer (1.5 litres per minute). In addition, such a combination of low flow rate compressor with accumulator, in conjunction with the dispensing system according to the invention, provides a dosing apparatus which is more lightweight, compact and readily portable than known dosing apparatuses.

With regard to the manifold, if the dispensing device according to the invention is used in conjunction with a low flow rate compressor, it is only necessary for the valve to switch the compressed air on and off to the nebulizer, in which case the manifold may only need to direct the compressed air in one direction only. However, if the dispensing system is used in conjunction with a conventional air supply such as a six litre per minute compressor or gas bottle supply, the manifold in conjunction with the valve directs the flow to either the nebulizer or the outlet orifice. In such circumstances, the valve in conjunction with the manifold has either one port or two outlet ports, depending on the application.

An important feature of the dispensing systems according to the invention lies in the volume of the tube linking the manifold and the port being less than 0.7 ml, preferably less than 0.5 ml. It has been found that by using lengths of tube which have a relatively small volume, the nebulizer starts to work as quickly as possible once the patient's inhalation has been detected. Typically, it is possible that the nebulizer can work within 50 milliseconds of detecting the patient's inhalation. To facilitate both the rapid response time and the low volume of the tube linking the manifold and the port, it is preferred that the valve is physically close to the nebulizer.

If a relatively long tube, or one with a large internal volume is used between the manifold and the nebulizer, this tube has to be pressurized before the nebulizer starts to operate. This can have significant effects on the performance of the system with regard to the controlling the rate of output of the nebulizer, since the nebulizer should preferably be provided with an essentially constant rate ("square wave") of pressure over time, so that its output is constant over time. The nebulizer used in the dispensing system is preferably one which delivers inspiratory patterns between 0.1 and 1.5 seconds duration.

The invention will now be described further by way of example only with reference to the accompanying drawings, in which:

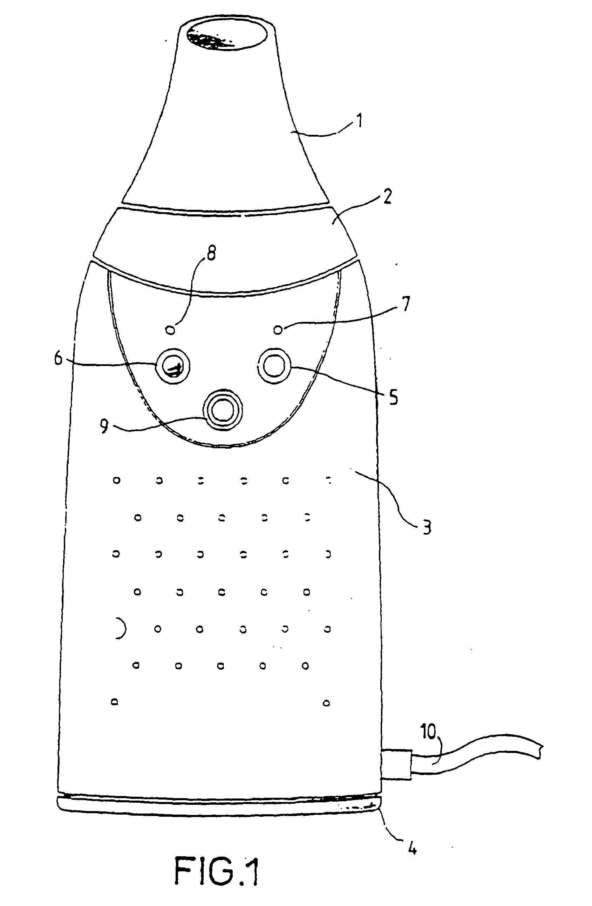

Figure 1 shows a schematic view of a dispensing system according to the invention, complete with a nebulizer and mouthpiece fitted;
Figure 2 shows a schematic cross-section view of the nebulizer/mouthpiece end of the dispensing system of Figure 1;
Figure 3 shows a further schematic cross-section view of the nebulizer/mouthpiece end of the dispensing system of Figure 1 from an orthogonal position to that shown in Figure 2;
Figure 4 shows a schematic cross-section view of the control system end of the dispensing system of Figure 1;
Figure 5 shows a schematic view of the dispensing system of Figure 1 attached to a low flow rate compressor;
Figure 6 shows a schematic representation of the combined dispensing system/compressor system of Figure 5;
Figure 7 shows a loading regime for loading an embodiment of dispensing system according to an aspect of the invention; and
Figure 8 shows cleaning regime for cleaning an embodiment of dispensing system according to an aspect of the invention.
Figure 9 shows an accumulator consisting of a generally hemispherical elastic diaphragm.
Figure 10 is a graph showing the nebulizer output rate of a typical nebulizer against the inspirator flow of a patient.

Referring to the figures, an embodiment of dispensing system according to the invention comprises a hand held dosimeter having a height of approximately 200 mm and a weight of approximately 200g. The dosimeter has a mouthpiece (1) and a nebulizer (2) attached, and a body (3) which contains a control valve, electronic circuitry and a battery. These components can be accessed through base (4).

The dosimeter is connected to a compressed air source via tube (10). To operate the dosimeter, the patient removes mouthpiece (1), and pours a liquid form of the medication (which be a liquid or a powder in a fluidised form, or any other similar form) into the nebulizer (2). Then, the dosimeter is connected to the compressed air supply via tube (10). By means of a switch device to be described later, the presence of a positive pressure in the dosimeter activates the control circuitry in the dosimeter, and switches it on.

In this embodiment, although the nebulizer has only one well in which the medication sits, the dosimeter is nevertheless pre-programmed to deliver the correct dose of two different nominated drugs. The dosimeter could, however, be constructed and pre-programmed so as to deliver any desired number of nominated drugs. The drug which has been loaded into the nebulizer is selected using selector buttons (5) and (6). Once the drug type is selected, LED's (7) and (8) indicate the drug type selected by the patient. Button (9) is a re-set button, so that the user can correct any errors in selection.

The nebulizer used in the dispensing system according to the invention can be any suitable nebulizer design with a known calibration constant which is used to nebulize substances such as medicaments, which is suitably adapted to fit the dispensing system, and calibrated with the dosimeter. Suitable nebulizers include those which use a source of compressed air to nebulize the medicament, and are, for example, described in European Patent No. 672,266 (Medic-Aid Limited), the contents of which are incorporated by reference.

When the drug has been selected, the patient breathes in through mouthpiece (1). A pressure sensor (to be further described later) within body (3) detects a pressure drop within the mouthpiece (1) due to the patient's inhalation, and then delivers a pre-programme pulse of nebulized medicament into the first 50% of the inspiratory profile, until the dose regime programmed into the dispensing system has been delivered.

The dose is calculated from a known rate of output against time for the drug selected, and the sum of all the nebulizer pulses which the dosimeter has delivered. Further information on how the doses of drug may be derived and pre-programmed into the dosimeter may be obtained from GB 2,294,402 (Medic-Aid Limited et al), the contents of which are incorporated herein by reference. In this instance, clinical trials have been conducted to determine the dose of drug that must be delivered to achieve the correct therapeutic effect, and this dose has been programmed into the dosimeter for the two nominated drugs.

When the programmed dose has been delivered, the LED adjacent the button relating to the selected drug flashes rapidly and a buzzer sounds, indicating that the treatment is finished, and thereby ensuing that a precise dosage of drug is delivered to the patient on every occasion.

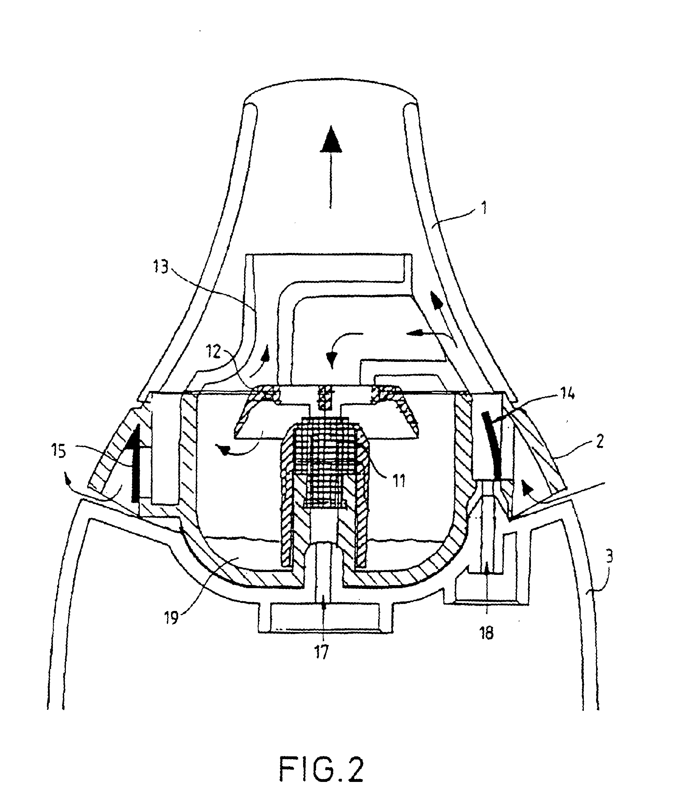

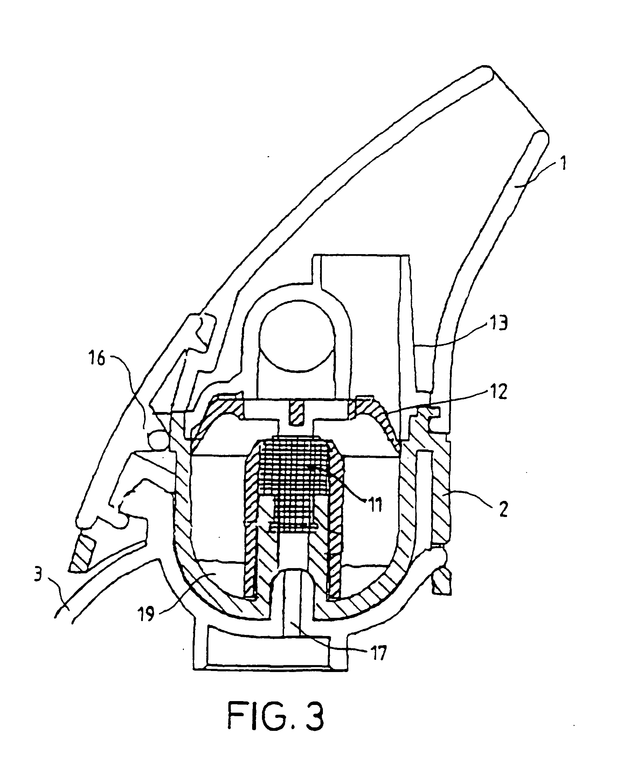

The nebulizer used in this embodiment is shown in more detail in Figures 2 and 3. Compressed air is supplied via port (17) to the nebulizer jet (11), and this works in conjunction with baffle (12) to aerosolize the liquid drug which has been placed in nebulizer bowl (19).

This nebulizer is venturi nebulizer which draws in air through the spout (13) into the centre of the baffle (12), and then up and out through the outlet of the spout (13) into the mouthpiece. As the nebulizer only generates aerosol during inspiration, the patients inhaled air is drawn through valve (14), and further through the mouthpiece to the patient. A small proportion of this air is drawn down through the centre of the nebulizer to operate the venturi system. If the flow through the nebulizer is completely independent of the patient's flow, the nebulizer produces a constant rate of output. Valve (14) creates a pressure drop within the mouthpiece, and this is monitored through port (18). On exhalation valve (14) will close, and the patient will exhale through valve (15). Further information as the nebulizer on the nebulizer is contained in EP 627,266 (Medic-Aid Limited).

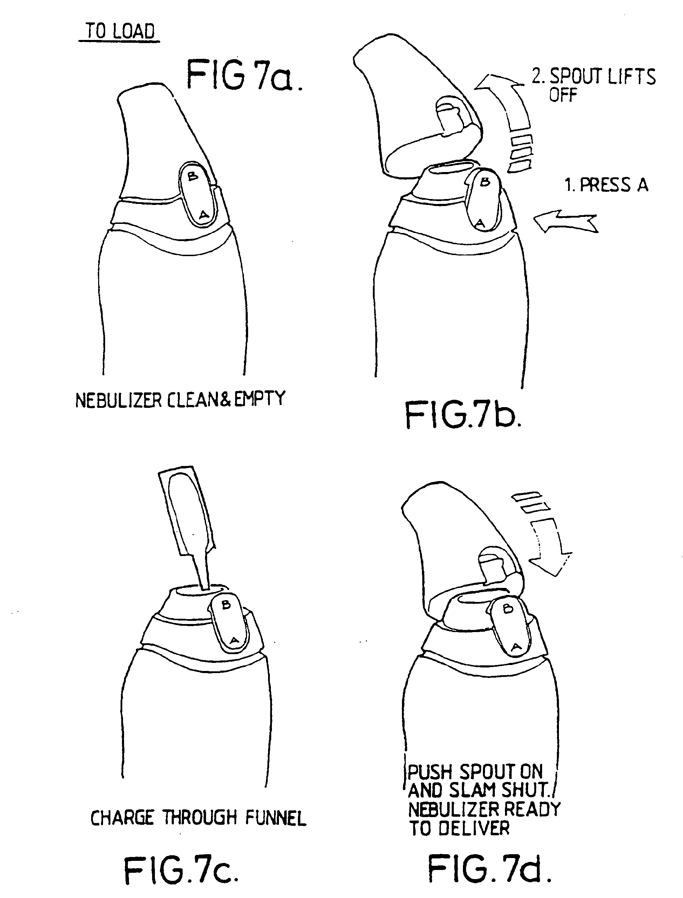

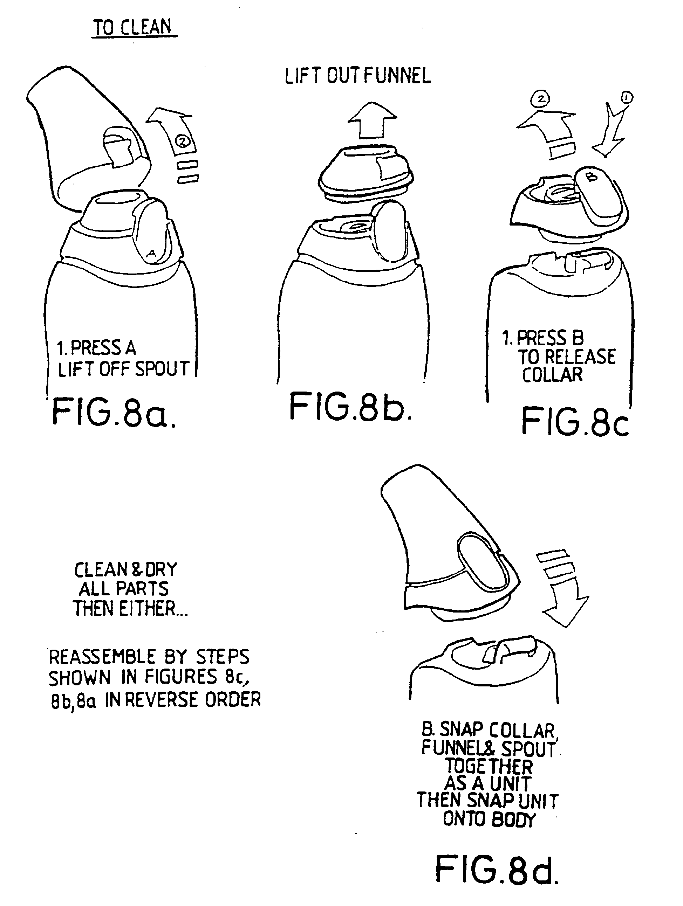

The complete assembly is held in place by catch (16). When the lower end of the catch is displaced the mouthpiece can be removed. The top end of the catch can then be tilted down to release the nebulizer bowl assembly from the main dosimeter case (3). This allows the whole nebulizer unit to be removed after the treatment has been completed, and cleaned completely separate from the main dosimeter case. This is further graphically illustrated in Figures 7 and 8.

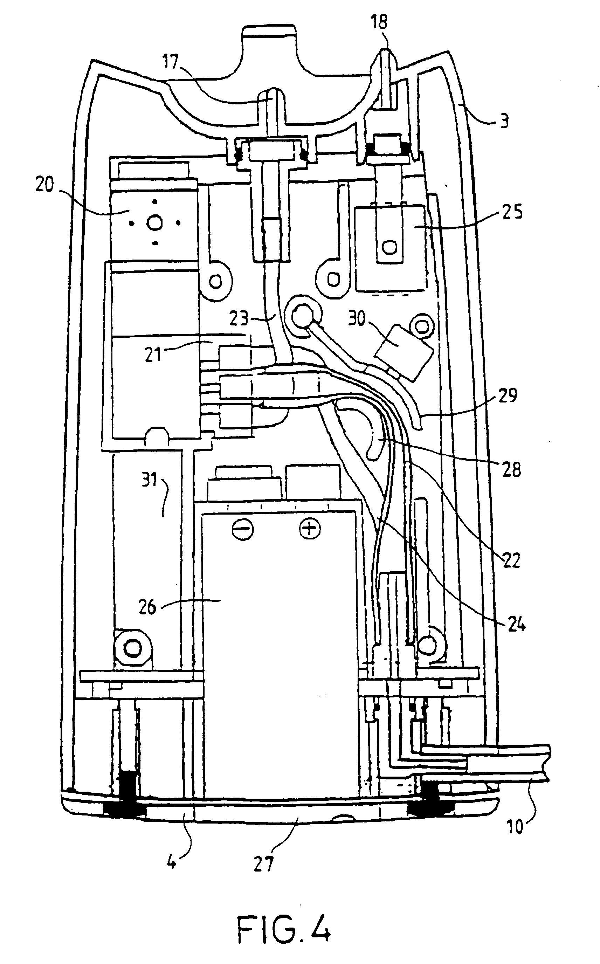

Figure 4 shows an internal section through the dosimeter, in particular showing the electronic configuration of the device. Air enters the dosimeter via tube (10) and through flexible tube (22) to the manifold (21) and onwards to valve (20). To determine whether there is pressure in the tube (22), it is configured around wall (28). When there is no pressure in the tube, the tube collapses. However, under pressure it expands, and moves lever (29) against switch (30), which switches the dosimeter on.

The dosimeter can be operated from various different compressed air sources; firstly it can use either a continuous air supply of approximately 6 litres per minute, generated from a conventional air compressor, or a compressed bottled gas supply such as is used in hospitals. With these compressed air sources, when the compressed air is not being supplied to the nebulizer via tube (23) and port (17), it must be vented externally through tube (24) out of the base of the dosimeter via an orifice which matches the size of the jet (11) diameter in the nebulizer. This maintains a constant pressure in the system irrespective of whether the valve is directing air to the nebulizer or the vent.

In an alternative and preferred embodiment, which can be used in conjunction with other forms of dosimeter, the dosimeter can be operated in conjunction with a low flow rate compressor system having a flow rate of 1-2 litres per minute, which has a fitted accumulator.

This compressor system generates a low flow of, for example, 1.5 litres per minute in the accumulator, and this allows the dosimeter to produce pulses of compressed air to the nebulizer with an equivalent flow rate of around 6 litres per minute, where the flow from the compressor is matched to the mean flow through the nebulizer (1.5 litres per minute). In this configuration tube (24) is sealed, and when the valve (20) is closed the pressure in the supply tube (10) is diverted into the compressor's accumulator. Port (18) connects the pressure sensor (25) to the mouthpiece. This is attached to the printed circuit board (31) which is powered by battery (26). When the electronic system detects the patient has inhaled, valve (20) diverts the pressure flow into tube (23) and out to a nebulizer via port (17). The battery is inserted into the dosimeter via door (27) into base (4).

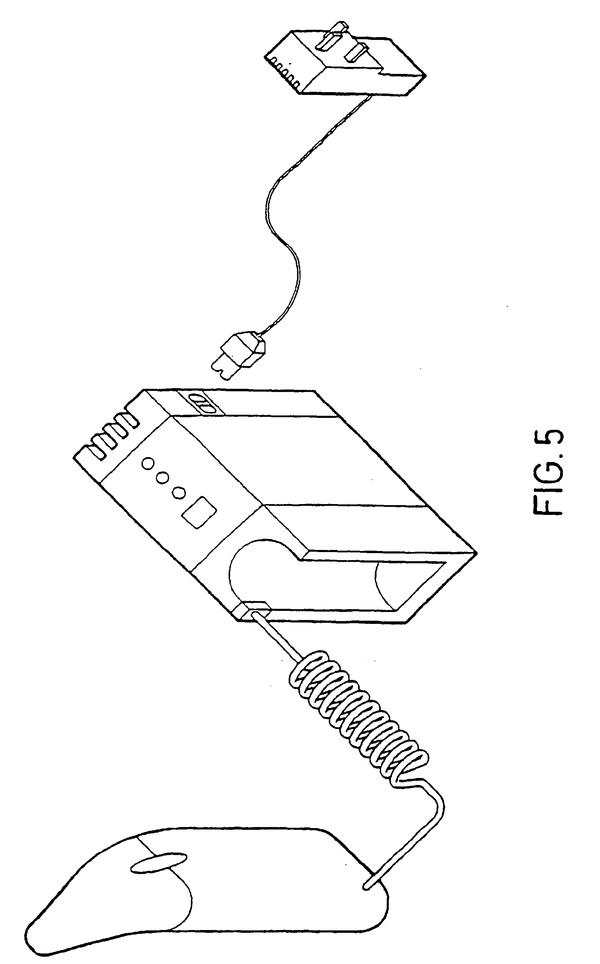

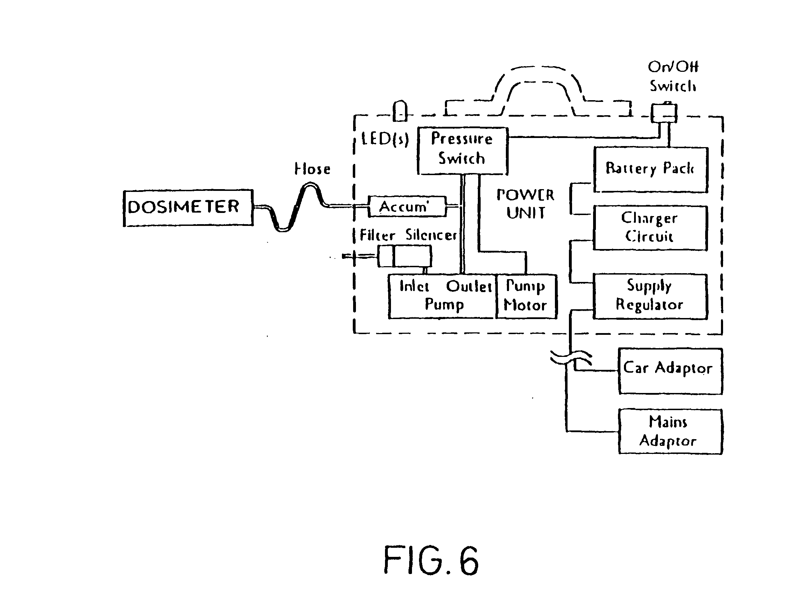

An example of a suitable low flow rate compressor which can be used as described above is shown in Figures 5 and 6, and comprises a small air compressor with a flow of approximately 1.5 litres per minute, and an accumulator with a volume of about 0.2 litres, which may be formed, for example, in the tubing between the compressor and the dosimeter, or using a bellows and spring arrangement within the compressor casing. Below is described a rubber hemisphere accumulator which works particularly well. The compressor operates until the accumulator has reached its capacity, and then switches off. The dosimeter receives pulses of air from the accumulator until the accumulator has been emptied to a specified volume (0.1 litre). The compressor then cuts in and refills the accumulator. The compressor produces a mean output flow rate which is known to the dosimeter, and the dosimeter controls its supply of air so that it does not exceed the mean flow rate from the compressor, and any one pulse delivered by the dosimeter does not exceed accumulator capacity. This system therefore utilizes the compressor, which is approximately one-quarter of the size of a conventional compressor system, and one quarter of the energy requirement. Such a compressor is also cheaper to manufacture and provide.

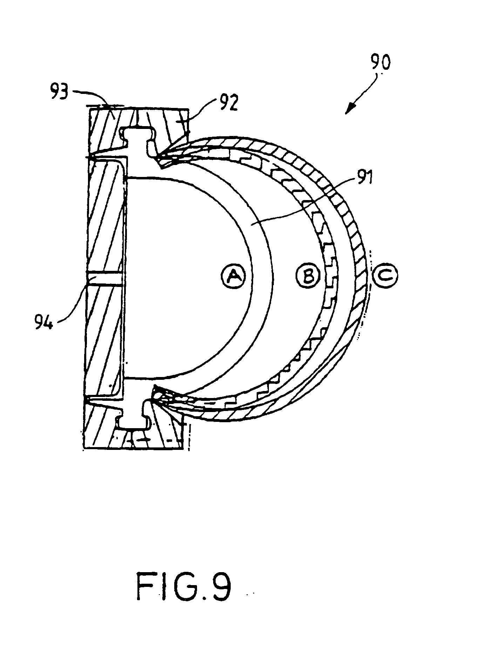

The accumulator in this embodiment should have a linear pressure to volume relationship over the period of a pulse delivery, which is typically 1.5 seconds long, with a nebulizer jet flow of 6 litres per minute, the accumulator should provide a volume flow of 150 millilitres at a constant pressure (lbar +/- 10%). It is difficult to obtain a steady linear pressure to volume relationship. Figure 9 shows a natural rubber hemispherical accumulator. The rubber has a linear load to extension between an extension of 100% and 500%. A diaphragm 91 is a hemispherical component which is held trapped between two rings 92 and 93. In Figure 9, the diaphragm is shown in three positions labelled A, B, C. Position A is the unloaded position from which the diaphragm starts and as compressed air is supplied via a port 94, the diaphragm expands to position C with a 200% extension. The compressed air supply is then stopped either by the actuation of a microswitch on the diaphragm surface (not shown) which stops the compressors, or by a pneumatic microswitch which then vents the compressor output. The diaphragm 91 then stays in this position until the valve sends a pulse of air to the nebulizer. When the diaphragm delivers air, the pressure drop during operation is less than 5%. The diaphragm can supply air at a stable pressure until it reaches position B which is a 120% extension. The microswitch would then restart the compressor, or the pneumatic microswitch would be closed. This hemisphere arrangement is relatively simple to manufacture, but has great advantages over other accumulator systems which might be used.

A suitable electrical configuration for such a compressor is shown in Figure 6.

For the dispensing system according to the invention to work effectively, the dosimeter valve (20) should be in close proximity to the nebulizer, so that when the system detects the patient's inhalation, the nebulizer starts to work as quickly as possible, typically in less than 50 milliseconds. This means that the length of tube (23) between the manifold outlet and the nebulizer jet must be short, with an internal volume less than 0.7 ml, preferably less than 0.5 ml, which represents 5% of the shortest pulse the nebulizer delivers. If a long tube is used between the valve and the nebulizer, this tube has to be pressurized before the nebulizer starts to operate. This significantly affects the performance of the system, as to control the rate of output of the nebulizer it must be supplied with a "square wave" of air pressure, so that the output is constant over time, and is delivered at the start of inhalation. The nebulizer delivers pulses to inspiratory patterns between 0.1 and 1.5 seconds' duration.

One of the simplest ways of determining the dose of medicament which is received by the patient is, as described above, to multiply the nebulizer output rate by the duration of each pulse. The doses then ascertained by summing the amount of medicament which is received during each pulse. This calculation relies on the fact that the output rate of a nebulizer should be constant regardless of the rate of inhalation of the patient. Therefore, provided that the nebulizer output rate is the same for a person who inhales slowly as for a patient who inhales quickly, the calculation will be accurate. If the nebulizer output rate varies with the speed of inhalation, the precision of the dosimeter will vary.

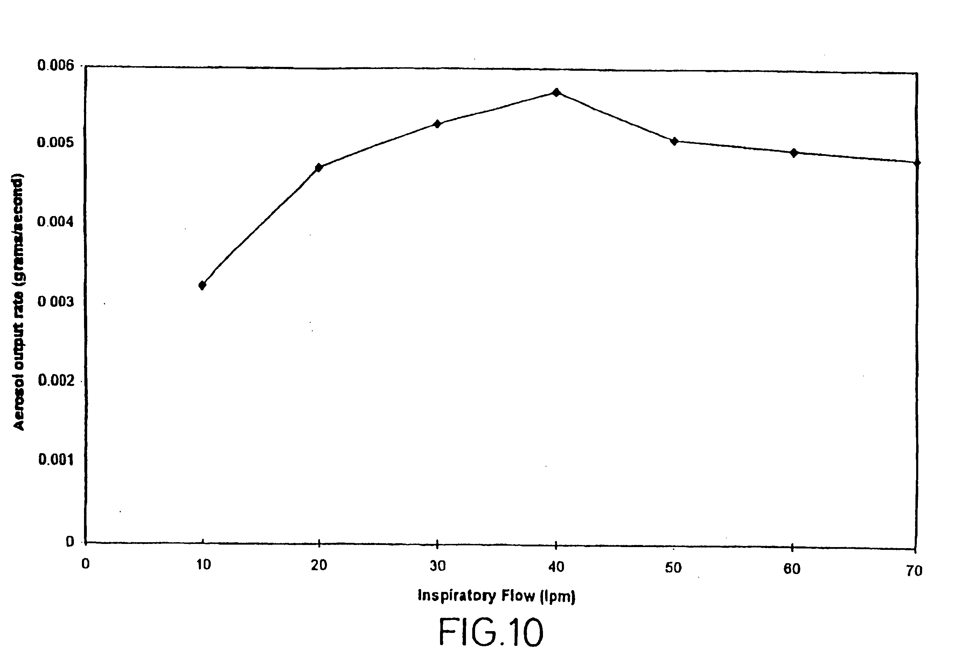

Figure 10 is a graph showing the variation of aerosol output rate with the speed of inspiratory flow for a typical nebulizer. As will be seen, the output is not constant.

It is therefore proposed to use the pressure sensor to provide information on the patient flow rate so that the correct nebulizer calibration rate is determined during patient inhalation. This may be done in the form of a look-up table which is quite effective, and the look-up table can have two or more calibration points as required to provide the necessary accuracy. A satisfactory look-up table can be achieved by using an approximation of the look-up table which, in the case of the graph shown in Figure 10 can be made up of two straight lines, one line generally following the curve to the 30 lpm point and a second line which generally follows the curve above that. Such an approximation works well because, in reality, the breathing pattern of a patient is not at a fixed level, but is continually changing.

Another way of using the correct calibration value is to use the computer which takes the inspiratory flow rate into account. One calibration value can be used above the 30 litres per minute level, and when the flow is below about 15 litres per minute, then the calibration constant is reduced to 60% of that value. Thus, the calibration may be achieved in a number of different ways not just a multi-point look-up table.

## Claims

1. A product dispensing system comprising a calibrated nebuliser (2) having a nebuliser jet (11), a mouthpiece (1), means (10,22) for sourcing compressed air, a manifold (21) for distributing the compressed air in at least one direction, a port (17), and a valve means (20) for controlling the manifold (21) and thereby the flow of compressed air to the port (17), the manifold (21) and the port (17) being linked by a length of tube (23), **characterised by** a nebuliser accommodation means which is accessed by the port (19), by a sensor for detecting a patient's breathing to indicate the rate of inhalation of the patient and by means for determining the dose dispensed, the dose determined on the basis of the output rate of the medicament of the nebuliser (21), and the duration of the delivery of the medicament, the dose determining means serving to adjust the value of the output rate used in calculating the dose on the basis of the rate of inhalation of the patient detected by the sensor.

2. A system according to claim 1 wherein the internal volume of the tube from the manifold outlet to the nebuliser jet is less than 0.7 ml.

3. A dispensing system as claimed in claim 1 or claim 2, pre-programmed to dose one or more medicaments according to predetermined dosage profiles.

4. A dispensing system as claimed in any one of claims 1 - 3, wherein the manifold is integral with the valve means.

5. A system according to any one of the preceding claims, wherein the sensor for detecting a patient's breathing is a pressure sensor for detecting a drop in pressure in the apparatus in response to a patient's breathing to indicate the rate of inhalation of the patient.

6. A dispensing system as claimed in any one of the preceding claims, additionally comprising a compressor having a flow rate of 1-2 litres per minute and an accumulator.

7. A dispensing system as claimed in any one of claims 1 to 6, additionally comprising a switch means which is responsive as to whether compressed air is flowing in the system.

8. A dispensing system according to any one of the preceding claims, where the manifold is a shaped block with integral galleys the presence of which allows the tubes to be connected to the ports on the valve.

9. A dispensing system according to any one of claims 1 to 5, further comprising a compressor and an accumulator.

10. A dispensing system according to claim 9, further comprising a lightweight compressor system and an accumulator.

11. A dispensing system according to claim 10, wherein the lightweight compressor generates a low flow rate of the order of 1 to 2 litres per minute.

12. A dispensing system according to claim 10 to 11, wherein the combination of the compressor and the accumulator allows the dispensing system to produce pulses of compressed air to the nebuliser with a flow of around of 6 litres per minute.

13. A dispensing system according to any one of claims 9 to 12, wherein the valve means serves to switch the compressed air on and off.

14. A dispensing system according to any one of claims 9 to 13, wherein the accumulator includes a resilient elastic body having a generally linear pressure to volume relationship.

15. A dispensing system according to claim 14, wherein the resilient body is a generally hemispherical resilient element.

16. A dispensing system according to claim 15, wherein the hemispherical element is a diaphragm extendable to 500% of its unloaded volume.

17. A dispensing system according to any one of the preceding claims wherein the manifold together with the valve means serve to direct the flow of compressed air either to the nebuliser or to an outlet orifice.

18. A dispensing system according to any one of the preceding claims, wherein the volume of the tube linking the manifold outlet to the nebuliser jet is less than 0.5 ml.

19. A dispensing system according to any one of the preceding claims wherein the nebuliser serves to deliver inspiratory pulses between 0.1 and 1.5 seconds in duration.

20. A dispensing system according to any one of the preceding claims, further comprising means for calculating the dosage of medicament on the basis of the known rate of medicament output against time for the selected drug.

21. A system according to any one of the preceding claims, further comprising an indicator for indicating when the require dose of the product has been delivered.

22. A system according to any one of the preceding claims, wherein the dose determining means includes a look-up table by which the output rate used in calculating the dose is based on the rate of inhalation of the patient.

23. A system according to claim 22, wherein the look-up table includes two or more calibration points.

24. A system according to any one of claims 1 to 21, wherein the dose determining means includes a computer arranged to use a first calibration value above 30 litres per minute rate at inhalation, and a second calibration point when the rate is below 15 litres per minute.

25. A system according to claim 24, wherein the second calibration point is 60% of the first.

## Patentansprüche

1. Eine Produktabgabevorrichtung, aufweisend einen kalibrierten Zerstäuber (2) mit einer Zerstäuberdüse (11), ein Mundstück (1), Mittel (10, 22) zur Lieferung von komprimierter Luft, einen Verteiler (21) zur Verteilung der komprimierten Luft in wenigstens eine Richtung, einen Auslass (17) und Ventilmittel (20) zur Steuerung des Verteilers (21) und damit des Flusses von komprimierter Luft zu dem Auslass (17), wobei der Verteiler (21) und der Auslass (17) durch eine Länge einer Röhre (23) verbunden sind, **gekennzeichnet durch** Zerstäuberaufnahmemittel, auf die **durch** den Auslass zugreifbar ist, **durch** einen Sensor zur Erkennung des Atems eines Patienten, um die Inhalationsrate des Patienten anzuzeigen und **durch** Mittel zur Bestimmung der abzugebenden Dosis, wobei die Dosis auf der Grundlage der Ausgaberate des Medikaments **durch** den Zerstäuber (21) und **durch** die Dauer der Medikamentenzufuhr bestimmt wird, wobei die Dosierungsbestimmungsmittel dazu dienen, den Wert der Ausgaberate, der bei der Berechnung der Dosis verwendet wird, auf der Grundlage der Inhalationsrate des Patienten, die **durch** den Sensor erkannt wird, einzustellen.

2. Eine Abgabevorrichtung nach Anspruch 1, bei der das Innenvolumen der Röhre von dem Verteilerauslass zur Zerstäuberdüse geringer als 0,7 ml ist.

3. Eine Abgabevorrichtung nach Anspruch 1 oder 2, vorprogrammiert zur Dosierung von einem oder mehreren Medikamenten gemäß bestimmten Dosierungsprofilen.

4. Eine Abgabevorrichtung nach einem der Ansprüche 1-3, bei der der Verteiler einstückig mit den Ventilmitteln ist.

5. Eine Abgabevorrichtung nach einem der vorhergehenden Ansprüche, wobei der Sensor zur Erkennung einer Atmung des Patienten ein Drucksensor ist, um einen Druckabfall in der Vorrichtung in Antwort auf den Atem eines Patienten zu erkennen, um die Inhalationsrate des Patienten anzuzeigen.

6. Eine Abgabevorrichtung nach einem der vorhergehenden Ansprüche, zusätzlich mit einem Kompressor mit einer Flussrate von 1-2 Liter pro Minute und einem Sammler.

7. Eine Abgabevorrichtung nach einem der Ansprüche 1-6, zusätzlich mit Schaltmitteln, die darauf ansprechen, ob komprimierte Luft in der Vorrichtung fließt.

8. Eine Abgabevorrichtung nach einem der vorhergehenden Ansprüche, bei der der Verteiler blockförmig mit einstückigen Führungen ist, deren Vorhandensein ermöglicht, dass die Leitungen mit in den Ausgängen am Ventil verbindbar sind.

9. Eine Abgabevorrichtung nach einem der Ansprüche 1-5, weiterhin mit einem Kompressor und einem Sammler.

10. Eine Abgabevorrichtung nach Anspruch 9, weiterhin mit einem leichtgewichtigen Kompressorsystem und einem Sammler.

11. Eine Abgabevorrichtung nach Anspruch 10, wobei der leichtgewichtige Kompressor eine niedrige Flussrate in der Größenordnung von 1 bis 2 Litern pro Minute erzeugt.

12. Eine Abgabevorrichtung nach Anspruch 10 oder 11, wobei die Kombination aus Kompressor und Sammler erlaubt, dass die Abgabevorrichtung Pulse von komprimierter Luft an den Zerstäuber mit einem Fluss von ungefähr 6 Litern pro Minute erzeugt.

13. Eine Abgabevorrichtung nach einem der Ansprüche 9-12, wobei die Ventilmittel dazu dienen, die komprimierte Luft ein- und auszuschalten.

14. Eine Abgabevorrichtung nach einem der Ansprüche 9-13, wobei der Sammler einen elastisch nachgiebigen Körper mit einem im wesentlichen linearen Verhältnis von Druck zu Volumen enthält.

15. Eine Abgabevorrichtung nach Anspruch 14, wobei der nachgiebige Körper ein im wesentlichen halbkugelförmiges nachgiebiges Element ist.

16. Eine Abgabevorrichtung nach Anspruch 15, wobei das halbkugelförmige Element eine Membran ist, die auf 500% ihres unbelasteten Volumens aufweitbar ist.

17. Eine Abgabevorrichtung nach einem der vorhergehenden Ansprüche, wobei der Verteiler zusammen mit den Ventilmitteln dazu dient, den Fluss von komprimierter Luft entweder zum Zerstäuber oder zu einer Auslassöffnung zu richten.

18. Eine Abgabevorrichtung nach einem der vorhergehenden Ansprüche, wobei das Volumen der Röhre, die den Verteilerauslass mit dem Zerstäuber verbindet, geringer als 0,5 ml ist.

19. Eine Abgabevorrichtung nach einem der vorhergehenden Ansprüche, wobei der Zerstäuber dazu dient, Inspirationspulse zwischen 0,1 und 1,5 Sekunden Dauer zu liefern.

20. Eine Abgabevorrichtung nach einem der vorhergehenden Ansprüche, weiterhin mit Mitteln zur Berechnung zur Dosierung des Medikaments auf der Grundlage der bekannten Medikamentausgaberate gegenüber der Zeit für den ausgewählten Wirkstoff.

21. Eine Abgabevorrichtung nach einem der vorhergehenden Ansprüche, weiterhin mit einer Anzeige zur Anzeige, wann die benötigte Dosis des Produkts geliefert wurde.

22. Eine Abgabevorrichtung nach einem der vorhergehenden Ansprüche, wobei die Dosisbestimmungsmittel eine Nachschlagetabelle enthalten, mittels der die Ausgangsrate zur Verwendung bei der Berechnung der Dosierung auf der Inhalationsrate des Patienten basiert.

23. Eine Abgabevorrichtung nach Anspruch 22, wobei die Nachschlagetabelle zwei oder mehr Kalibrierpunkte aufweist.

24. Eine Abgabevorrichtung nach einem der Ansprüche 1-21, wobei die Dosierungsbestimmungsmittel einen Computer aufweisen, der einen ersten Kalibrierwert über 30 Liter pro Minute Inhalationsrate und einen zweiten Kalibrierungspunkt zu verwenden vermag, wenn die Rate unter 15 Liter pro Minute ist.

25. Eine Abgabevorrichtung nach Anspruch 24, wobei der zweite Kalibrierpunkt 60% des ersten beträgt.

## Revendications

1. Système distributeur de produits, comprenant un nébuliseur étalonné (2) comportant un ajutage de nébuliseur (11), une embouchure (1), des moyens (10, 22) destinés à servir de source d'air comprimé, une tubulure (21) destinée à distribuer l'air comprimé dans au moins une direction, un port (17), et un moyen de vanne (20) pour contrôler la tubulure (21) et donc le débit d'air comprimé arrivant au port (17), la tubulure (21) et le port (17) étant reliés par une certaine longueur de tube (23),
**caractérisé par**
- un moyen d'adaptation de nébuliseur auquel on accède par le port (19);
- un capteur destiné à détecter la respiration d'un patient pour indiquer le taux d'inhalation du patient ; et
- un moyen pour déterminer la dose distribuée, cette dose étant déterminée sur la base du débit de sortie de médicament du nébuliseur (21), et de la durée de distribution du médicament, le moyen de détermination d'une dose servant à régler la valeur du débit de sortie utilisée pour calculer la dose sur la base du taux d'inhalation du patient qui est détecté par le capteur.

2. Système selon la revendication 1,
dans lequel
le volume intérieur du tube entre la sortie de la tubulure et l'ajutage du nébuliseur, est inférieur à 0,7 ml.

3. Système distributeur selon la revendication 1 ou 2,
**caractérisé en ce qu'**
il est préprogrammé pour doser un ou plusieurs médicaments suivant des profils de dosage prédéterminés.

4. Système distributeur selon l'une quelconque des revendications 1 à 3,
dans lequel
la tubulure fait partie intégrante du moyen de vanne.

5. Système selon l'une quelconque des revendications précédentes,
dans lequel
le capteur destiné à détecter la respiration d'un patient est un capteur de pression servant à détecter une chute de pression dans l'appareil en réponse à la respiration du patient, pour indiquer le taux d'inhalation de ce patient.

6. Système distributeur selon l'une quelconque des revendications précédentes,
comprenant en outre
un compresseur ayant un débit de 1 à 2 litres par minute, et un accumulateur.

7. Système distributeur selon l'une quelconque des revendications 1 à 6,
comprenant en outre
un commutateur répondant au fait que de l'air comprimé s'écoule ou non dans le système.

8. Système distributeur selon l'une quelconque des revendications précédentes,
dans lequel
la tubulure est un bloc mis en forme comportant des galeries intégrées dont la présence permet aux tubes d'être connectés aux ports de la vanne.

9. Système distributeur selon l'une quelconque des revendications 1 à 5,
comprenant en outre
un compresseur et un accumulateur.

10. Système distributeur selon la revendication 9,
comprenant en outre
un système de compresseur léger et un accumulateur.

11. Système distributeur selon la revendication 10,
dans lequel
le compresseur léger génère un débit faible de l'ordre de 1 à 2 litres par minute.

12. Système distributeur selon les revendications 10 et 11,
dans lequel
la combinaison du compresseur et de l'accumulateur permet au système distributeur de produire des impulsions d'air comprimé allant vers le nébuliseur avec un débit de l'ordre de 6 litres par minute.

13. Système distributeur selon l'une quelconque des revendications 9 à 12,
dans lequel
le moyen de vanne sert à commuter l'air comprimé en MARCHE/ARRET.

14. Système distributeur selon l'une quelconque des revendications 9 à 13,
dans lequel
l'accumulateur comprend un corps flexible élastique ayant une relation généralement linéaire entre la pression et le volume.

15. Système distributeur selon la revendication 14,
dans lequel
le corps élastique est un élément élastique généralement hémisphérique.

16. Système distributeur selon la revendication 15,
dans lequel
l'élément hémisphérique est un diaphragment extensible jusqu'à 500 % de son volume non chargé.

17. Système distributeur selon l'une quelconque des revendications précédentes,
dans lequel
la tubulure associée à la vanne sert à diriger le débit d'air comprimé soit vers le nébuliseur soit vers un orifice de sortie.

18. Système distributeur selon l'une quelconque des revendications précédentes,
dans lequel
le volume du tube reliant la sorite de la tubulure de l'ajutage de nébuliseur, est inférieur à 0,5 ml.

19. Système distributeur selon l'une quelconque des revendications précédentes,
dans lequel
le nébuliseur sert à délivrer des impulsions d'inspiration d'une durée comprise entre 0,1 et 1,5 seconde.

20. Système distributeur selon l'une quelconque des revendications précédentes,
comprenant en outre
un moyen pour calculer le dosage de médicament sur la base du début de sortie de médicament connu en fonction du temps, pour le produit pharmaceutique sélectionné.

21. Système distributeur selon l'une quelconque des revendications précédentes,
comprenant en outre
un indicateur destiné à indiquer quand la dose requise du produit a été délivrée.

22. Système distributeur selon l'une quelconque des revendications précédentes,
dans lequel
le moyen de détermination de dose comprend une table d'indicateur grâce à laquelle le débit de sortie utilisé pour calculer la dose est basé sur le taux d'inhalation du patient.

23. Système selon la revendication 22,
dans lequel
la table d'indicateur comprend deux ou plusieurs points d'étalonnage.

24. Système selon l'une quelconque des revendications 1 à 21,
dans lequel
le moyen de détermination de dose comprend un ordinateur disposé pour utiliser une première valeur d'étalonnage au-dessus d'un débit de 30litres par minute à l'inhalation, et un second point d'étalonnage lorsque le débit est au-dessous de 15 litres par minute.

25. Système selon la revendication 24,
dans lequel
le second point d'étalonnage est de 60 %du premier.
